# EUROPEAN PATENT APPLICATION

(11) **EP 0 911 319 A1**
(43) Date of publication of application: **28.04.1999**
(21) Application number: 97115086.7
(22) Date of filing: 01.09.1997
(51) Int. Cl.: C07C 259/06, A61K 31/185

(54) **Malonic acid based matrix metalloproteinase inhibitors**

(71) Applicant: Roche Diagnostics GmbH, 68298 Mannheim (DE)
(72) Inventor: The designation of the inventor has not yet been filed

(57) **Abstract**

The invention concerns compounds which are represented by the general formula I, 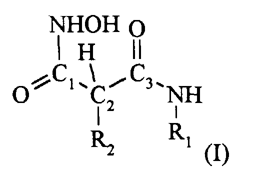 wherin the meaning of the substitutes is given in the claims,
their process of production and their use as MMP inhibitors.

## Description

The invention concerns new matrix metalloproteinase inhibitors which are based on the structure of malonic acid hydroxamates. The invention further concerns methods for the production of the inhibitors and their use, especially in the field of therapeutics.

Matrix Metalloproteinases (MMPs, Matrixins) comprise a family of Ca-containing Zn-endopeptidases, which exhibit proteolytic activities towards most if not all of the constituents of the extracellular matrix, such as the interstitial and basement membrane collagens, fibronectin and laminin. They play a pivotal role in normal tissue remodeling and are particularly implicated in other processes such as ovulation, embryonic growth and differentiation.

At least 16 different and yet highly homologous MMP species have been characterized, including the interstitial fibroblast collagenase (MMP-1, HFC), the neutrophil collagenase (MMP-8, HNC), two gelatinases, stromelysins (such as MMP-3 and MMP-10) and Matrilysin. These proteinases share a number of structural and functional features but differ somewhat in their substrate specificity.

All MMPs are secreted as multidomain proteolytically activable proenzymes with a ∼ 80 residue activation peptide which in most cases is followed by the ∼ 165 residue catalytic domain terminated by a ∼ 210 residue hemopexin-like domain. The catalytic domain contains a conserved HEXXHXXGXXH zinc binding sequence characteristic for the "metzincin" superfamily¹ and exhibits full activity towards most small peptide substrates.

MMPs are important for normal tissue development and remodeling and have been implicated in various disease processes such as tumor growth and metastasis, rheumatoid and osteoarthritis, periodentitis, corneal ulceration, arteriosclerosis and emphysema. Thus, inhibitors for MMPs could be used to treat these diseases. Three endogenous protein inhibitors (TIMP-I, II, III) which block the proteolytic activity of the MMPs in a more or less specific manner have been described to date. Virtually all specific synthetic MMP inhibitors designed so far are reversible peptidyl inhibitors which interact with the active site of their target enzyme. They contain a chelating group capable of interacting with the catalytic zinc (without removing it), such as a hydroxamate, thiol, carboxylate or phosphinic group, coupled with a peptidic moiety used for binding to the substrate recognition site of the enzyme. ² In this way the inhibitors are targeted towards and are specific for the desired zinc enzyme. To avoid or minimize side effects their is need for more active and/or more specific MMP inhibitors.

The invention concerns MMP inhibitors which bind to the MMPs in a manner completely different from the above-mentioned synthetic inhibitors. The inhibitors of the invention are somehow related only to the inhibitors of WO 96/17838, but are superior in their pharmacological properties. The compounds of the invention are optimized regarding the binding properties of the tail group (R₁) which binds in an optimized manner to the S1 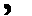 pocket of HNC. This is inter alia due to their hydrophobicity. In addition, the tail group is less sensitive to metabolic degradation compared with the examples disclosed in WO 96/17838 which contain at least one peptide group in their respective tail groups.

The new MMP inhibitors are compounds which are represented by the general formula I,

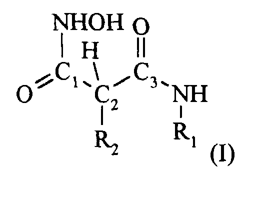

If the residue R1 of the tail group shows certain hydrophobicity values, improved inhibition of MMPs is observed. It is advantageous, if the first three backbone atoms adjacent to the C₃(O)NH structural element in the inhibitors of the invention consists of carbon atoms. A useful hydrophobicity parameter is the logP which can be calculated using 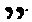 PCModels CLOGP3" provided by Daylight Chemical Information Systems, Inc (1993). The coefficients are based on Hansch, C. & Leo, A.: Substituent Constants for Correlation Analysis in Chemistry and Biology. Wiley Interscience New York (1979), the algorithm is based on Chou, J. & Jurs, P., J. Chem. Inf. Comput. Sci. 19, 172 (1979). The fragments are calculated as molecules H₂N-R1 and not as radicals or ions.

### Examples:

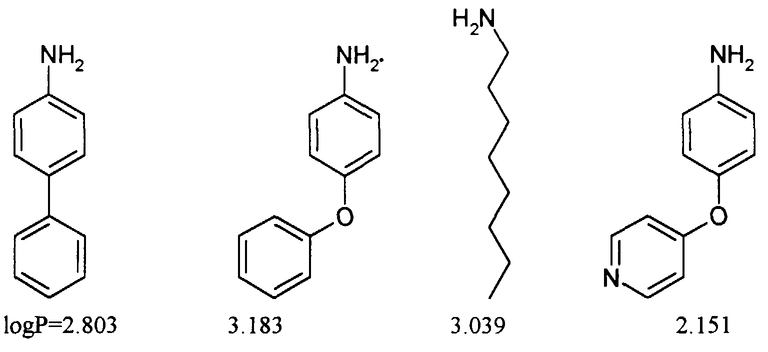

The logP-values of R1 in the compounds of the invention as calculated for H₂N-R1 show values between 1 to 4, preferred 1.5 to 3.5, more preferred between 2 and 3.2

Therefore new MMP inhibitors are compounds which are represented by the general formula I, 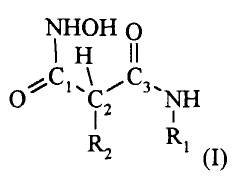 wherein
R₁ shows a logP-value between 1 and 4 if calculated as H₂N-R₁ with the proviso that no peptide groups are part of the chain and the proviso that at least the beginning three backbone atoms of the chain are carbon atoms.
R₂ is a residue of 2 to 10 backbone atoms, which interacts with the amino acid 161 of HNC, said residue being saturated or unsaturated, linear or branched, and contains optionally homocyclic or heterocyclic structures,

Alternatively R₁ can be defined as representing a chain which is optionally substituted once to four times and the chain consists of 7 to 11 backbone atoms comprising at least 7 C atoms with the proviso that no peptide groups are part of the chain and the proviso that at least the beginning three backbone atoms of the chain are carbon atoms.

The chain in R₁ contains preferably no ester group. Preferably, the number of substitutions of the chain is 2 or less and only one of the optional substitutes is within the first 3 backbone atoms.

More preferable R₁ is a residue which binds to the S1' pocket of HNC and is one of the following residues: 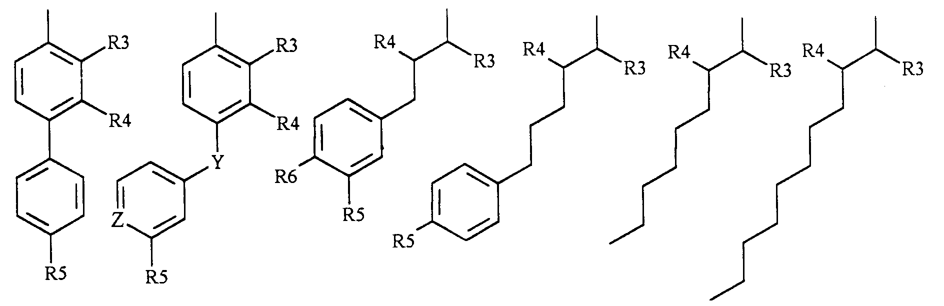 whereby
R3 and R4 independently have the meanings H, C₁-C₁₀-alkyl, aryl, arylalkyl, C₁-C₁₀-heteroalkyl, heteroaryl, heteroarylalkyl, heteroarylheteroalkyl, arylheteroalkyl,
R3 and R4 have in the case of a phenyl substitution in addition independently of each other the meaning of NR7R8
   R7 and R8 is independently of each other hydrogen or lower alkyl R3 and R4 have in the case of an alkyl substitution in addition independently of each other the meaning of SO₂R9 or COR10
   R9 is hydrogen or lower alkyl
   R10 is hydrogen, lower alkyl, or is one of the Cα-residues of an amino acid
R5 and R6 independently have the meanings H, OH, halogen, lower alkyl, lower alkenyl, or lower heteroalkyl, preferable at least one is hydrogen,
Y has the meanings O, S, NH, (CH₂)ₙ, n is 1, 2 or 3,
Z has the meanings N or CH.

Preferable at least one of R3 and R4 is hydrogen, more preferred R4 is hydrogen.

Preferable at least one of R5 and R6 is hydrogen, more preferred R6 is hydrogen.
R5 is preferably H if Y has the meaning (CH₂)₂ or (CH₂)₃,
R5 and R6 independently have the preferred meanings H, OH, Halogen, lower alkyl, or lower heteroalkyl,
Y is preferably O,

Highly preferred are the following residues for R₁: 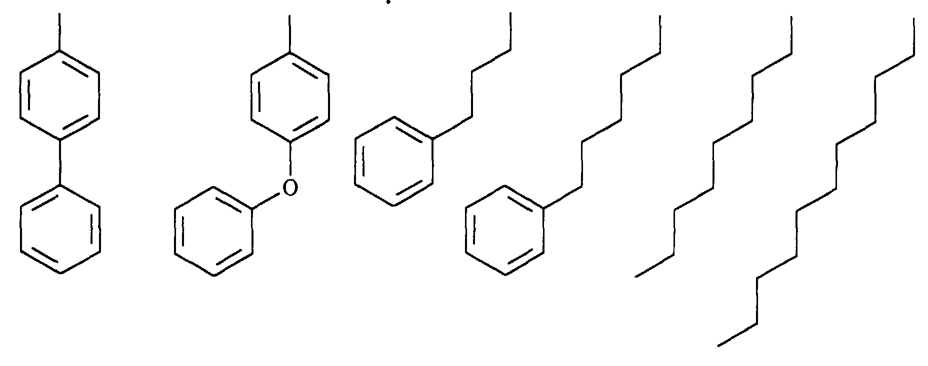 and within those residues especially preferred are the residues 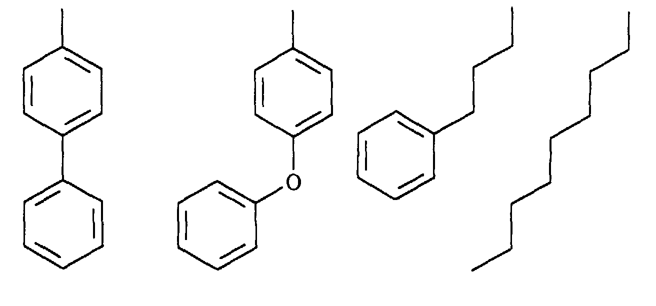
R2 is preferably isobutyl, phenyl, benzyl or homobenzyl

The compounds of the invention also comprise racemates, diastereomers, pharmacologically acceptable salts and prodrugs of compounds of general formula I. Examples for prodrugs are esters like ethyl or benzyl esters which are metabolized in vivo to the compounds of formula I.

Backbone atoms are C, N, O and S, hetero atoms denote N, O and S.

Peptide group denotes -C(O)-NH- which is bound to two adjacent C atoms.

Chain denotes in R1 for the longest linear backbone atom chain wherein rings if part of the chain count as maximally 4 backbone atoms. Rings per se as part of the chain do not represent substitutes. For examples the chain length of -(CH₂)₃-CH(CH₃)-CH₂-CH₃ equals 6 (1 Subst.), -(CH₂)₃-Ph(p-CH₂-CH₃) equals 9, -(CH₂)₃-Ph(m-CH₂-CH₃) equals 8 and -(CH₂)₃-Ph equals 7 (all 0 Subst.). If rings are part of the chain, only chains with one or more monocylic rings are claimed in the invention. Chains with bicyclic and highercylic ring systems are excluded from the invention.

Alkali salts, ammonium salts, trifluoroacetates or hydrochlorides are used above all as pharmacologically acceptable salts which are usually produced for example by titrating the compounds with inorganic or organic bases or acids such as e.g. sodium or potassium bicarbonate, sodium hydroxide solution, potassium hydroxide solution, aqueous ammonia or amines such as trimethylamine or triethylamine, trifluoroacetic acid or hydrochloric acid. The salts are usually purified by precipitation from water/acetone.

In all cases lower alkyl should represent a straight-chained or branched C₁-C₄ alkyl group such as e.g. methyl, ethyl, propyl, isopropyl, butyl or isobutyl, in particular methyl, ethyl, propyl, and isobutyl.

Lower alkenyl denotes unsaturated residues with 2,3 or 4 carbon atoms such as allyl, or but-2-enyl, but above all allyl.

Lower heteroalkyl denotes for an alkyl residue where 1, 2 or 3 C-atoms are substituted by heteroatoms. Preferably only one heteroatom is present which is most preferably O.

Halogen means fluorine, chlorine, bromine or iodine, preferably chlorine.

Homocyclic or heterocyclic ring denote for a saturated or unsaturated 5-6-membered ring such as a pyrrolidine, piperidine, morpholine or pyrroline ring.

The homocyclic and heterocyclic rings can be optionally substituted once or twice by C₁-C₆ alkyl groups, preferably a methyl, ethyl or isopropyl groups as well as by chlorine, bromine or hydroxy, methoxy, benzyloxy, amino, methylamino, dimethylamino or benzylamino groups.

Aryl usually denotes a phenyl residue which is optionally substituted once or several times.

Heteroaryl usually denotes a pyridine, pyridazine, pyrrole, thiophene, furan or imidazole ring which is substituted once or several times.

Bicyclic aryl usually denotes an indane or naphthalene residue which is optionally substituted once or several times, preferably a naphthalene residue.

Aryl, bicyclic aryl and hetaryl residues can be optionally substituted once or several times by C₁-C₆ alkyl groups, preferably a methyl, ethyl or isopropyl group as well as by chlorine, bromine, fluorine or hydroxy, alkoxy such as e.g. methoxy, benzyloxy, acetyloxy, carboxy, ethoxycarbonyl, aminocarbonyl, methylaminocarbonyl, dimethylaminocarbonyl, cyano, amino, methylamino, dimethylamino, benzylamino, acetylamino, benzoylamino and amidine groups.

Arylalkyl usually denotes an unsubstituted or once or several-fold substituted benzyl, phenethyl, phenyl-propyl, phenylbutyl or phenylpentyl residue, preferably a benzyl, phenethyl or phenylpentyl residue. C₁-C₆ alkyl residues, preferably a methyl, ethyl or isopropyl group as well as chlorine, bromine fluorine or hydroxy, methoxy, benzyloxy, acetyloxy, carboxy, ethoxycarbonyl, aminocarbonyl, methylaminocarbonyl, dimethylamino-carbonyl, cyano, amino, methylamino, dimethylamino, benzylamino, acetylamino, benzoylamino and amidino groups come into consideration as substitutes.

Arylheteroalkyl, heteroarylheteroalkyl and heteroarylalkyl denote for the same residues as arylakyl but there is at least one carbon substituted by a heteroatom.

C₁-C₁₀-alkyl and C₁-C₁₀-heteroalkyl, denote for a straight chained branched group with 1 to 10 backbone atoms.,

Amino acid denote for proteinogenic amino acids and non-proteinogenic amino acids. For D- and L-amino acids, preferred are proteinogenic amino acids. Examples for non-proteinogenic aminoacids are: 2-Amino-2-Methylbutanecarboxylic acid, 2-Fluoro-β-Alanine, β-Alanine, 2,3-Diaminosuccinic acid, β-Aminoisobutyr carboxylic acid, Isoserine, 2-Amino-3-Hydroxy-4-Methylpentanecarboxylic acid, 2-Amino-3-Methoxybutanecarboxylic acid, Diaminopropione acid, 2-Amino-2-Methyl-3-Hydroxypropanecarboxylic acid, 2-Amino-2-Methylbutanedicarboxylic acid, 2-Amino-3-Hydroxy-3-Methylbutanecarboxylic acid, 2,3-Diaminopropione acid, 2-Amino-2-Methyl-3-Hydroxypropanecarboxylic acid, 2-Amino-2-Methylbutanedicarboxylic acid, 2-Amino-2-Methyl-4-Pentenecarboxylic acid, 2-Amino-3-Methoxypropanecarboxylic acid, 1-Amino-1-Cyclohexanecarboxylic acid, 1-Amino-1-Cyclopentanecarboxylic acid, 1-Aminocyclobutanecarboxylic acid, 1-Aminocyclopropanecarboxylic acid, 2-(2-Furyl)-Glycine, 2-Amino-3-Fluorobutyr acid, 2-Aminoisobutyr acid, 3-Chloro-Alanine, 3-Fluoro-Norleucine, 3-Fluoro-Valine, 3-Fluoroalanine, 3-Methoxy-Valine, α-Cyano-Alanine, α-Methyl-Leucine, β-Chloro-Alanine, β-Cyano-Alanine, β-Hydroxy-Leucine, β-Hydroxyaspartic acid, 3-Hydroxyaspartic acid, 2-Aminobutyric acid, Allylglycine, γ-Methylleucine, Homoserine, Norleucine, Norvaline, tert-Leucine, 2,3-Diaminosuccinic acid, 2-Amino-4-Pentenecarboxylic acid, 2-Aminoheptanecarboxylic acid, 2-Cyclopropyl-2-Methylglycine, 4-Thiaisoleucine, Allothreonine, α-Methylaspartic acid, α-Methylserine, β-Hydroxynorvaline, β-Methylaspartic acid, Homocysteine, O-Methylserine, Penicillamine, Propargylglycine, Vinylglycine, H-4,5-Dehydro-Leu-OH, H-α-Me-Val-OH, H-Propargyl-Gly-OH, H-Allo-Ile-OH, H-Pra-OH, H-Trans-4,5-Dehydro-Lys-OH, 3-Hydroxyaspartic acid, 6-Hydroxynorleucine, Allo-Isoleucine, Allyl Glycine, α-Amino-N-Butyric acid, γ-Methylleucine, α,β-Diaminosuccinic acid, O-Carbamoyl-Serine, S-Methyl-Cysteine, Citrulline, Cyclohexylalanine, α,γ-Diaminobutyric acid, α,β-Diaminopropione acid, Methionine-Sulfoxide, C^{α}-Methyl-Alanine, N-Methyl-Glycine (Sarkosine), Naphtylalanine, Ornithine, 1,2,3,4-Tetrahydroisochinoline-3-carboxylic acid, Homocysteine, 4-Hydroxy-Proline, 5-Hydroxy-Lysine, Aminobutyric acid, Pantonine, Glucosamine acid, Lanthionine, Aliine, Dopa, Canavanine, Oletopine, β-Lysine, β-Alanine.

For example, the Cα-residue of Alanine is CH(NH₂)(CH₃).

The term "inhibition" according to the invention means a substantial reduction of MMP activity in vitro and in vivo. The MMP activity can be determined in vitro, for example, in an enzyme assay according to F. Grams (1993)³. "Substantial inhibition" means an inhibition of at least about 50%, preferably at less than mmolar concentration of the inhibitor (based on MMP activity without an inhibitor). Generally, an inhibition of more than about 80% to 90% is found.

The chelating group of the new inhibitors interacts with the catalytic zinc (which is situated at the bottom of the active site cleft in MMPs and is penta-coordinated by three histidines and by NHOH and O of the inhibitor) in a bidentate manner. The tail group of the inhibitors according to the invention adopts a bent conformation and inserts into the S1' pocket (subsite). In contrast to this, the tail group of most of the known inhibitors binds in an extended manner along the active site cleft (for definition of binding sites see ⁴).

The reason for the difference in binding between the new inhibitors and the inhibitors of the state of the art lies in a number of essential new structural features of the inhibitors according to the invention:

### A. The (pseudo)malonic acid basic structure.

The malonic acid structure defines three binding positions of the inhibitor. The chelating group binds via NHOH and O at C₁ as bidentate to the active site zinc in the MMPs.

The second binding site is defined by the interaction between R₂ and the amino acid 161 of the HNC. The term "interacting to amino acid 161" means the interaction with the surface area around amino acid 161, whereby preferably the binding to amino acid 161 is included. The binding derives, for example, from van der Waals or hydrophilic interaction.

A further binding of the malonic acid basic structure to MMPs is accomplished via the oxygen of C₃ at the tail group. This oxygen is hydrogen bonded to the amide proton of leucin 160 of HNC.

### B. The tail group

The inhibitor according to the invention binds, via R₁ to the S1' pocket. The pocket consists of:
human neutral collagenase (HNC):
L 193, V 194, H 197, E 188, L 214, Y 216, P 217, Y 219, A 220, R 22 amino acid (numbering according to Reinemer et al (1994)⁵).
HFC:
L 181, A 182, R 214, V 215, H 218, E 219, Y 237, P 238, S 239, Y 240 amino acid (numbering according to Lovejoy et al (1994)⁶).
Stromelysin:
L 197, V 198, H 201, E 202, L 218, Y 220, L 220, L 222, Y 223, H 224, S 225, A 226 amino acid (numbering according to Gooley et al (1994)⁷).

Therefore, an essential feature of the compounds is that in contrast to the MMP inhibitors according to the state of the art, the structure of the tail group is highly relevant for the inhibitory activity of the compounds according to the invention. The tail groups of the inhibitors according to the state of the art do not bind to the essential binding sites in the MMPs in an optimized manner. In the case of the compounds according to the invention, however, the binding of the tail group R₁ to the S1' pocket of the MMPs constitutes the essential part of the binding between the inhibitor and MMP and, consequently for the inhibitory activity. Thus it is essential that the tail group R₁ has a structure which fits well into the S1' pocket.

It has been found that residues pointing into S1 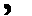 are favourable that have no larger extension from an axis matching the nitrogen connected to R1 and the last non-hydrogen atom in the chain of R1 than 3.2 Å, if said residue is in extended conformation. Preferrable the extension is less than 3.0 Å. This distance can be calculated by energy-minimising said residue without additional substituents with a standard molecular mechanics program like Discover/Insight II (MSI, San Diego) and a standard forcefield therein (e.g. CVFF). For the calculation the amino group is saturated with hydrogens, i.e. the whole residue is H₂N-R1. After minimisation a line can be drawn from the R1-attached nitrogen to the last non-hydrogen atom of the chain. Now the distance from this line to the atom (including hydrogen belonging to atoms of the chain), which is most far away, can be determined. Examples are:

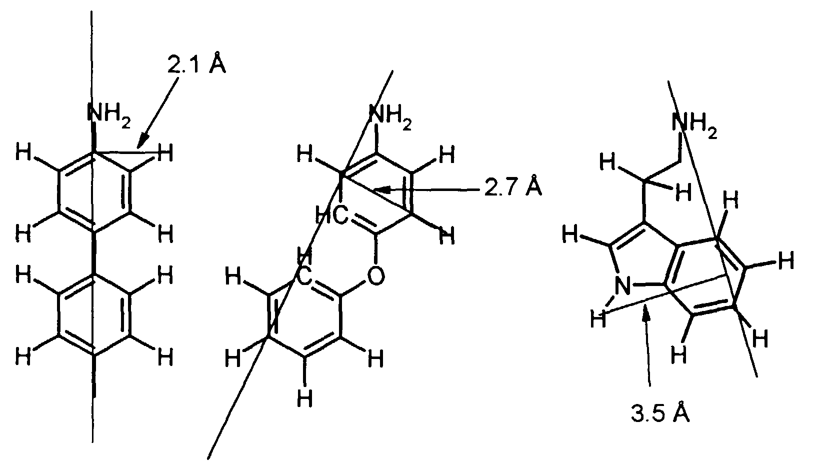

'Binds to the S1 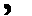 pocket' means in this invention that the maximal distance for an atom of the tail group or its connected hydrogen atom is 3.2 Å. I.e. residues like e.g. tryptophan are to bulky and are not claimed. The preferred distance is maximal 3.0 Å.

These requirements are fulfilled by synthetic compounds which contain a zinc chelating group which is spaced by one carbon from the substitute R₂ which constitutes an auxiliary binding site. It interacts with the surface of the protein with van der Waals and/or hydrophilic interactions. The tail group R₁ has to be designed for insertion into a pocket of the protein of well defined geometry and surface properties. In the upper part the environment is mainly hydrophobic, where hydrophobic interactions can be exploited, whereas the lower part contains also several hydrophilic sites, thus allowing for hydrogen bonding. Correspondingly hydrogen bond acceptors and donors are built-in in this portion of the inhibitor molecules.

Due to the short distance between the zinc binding region and O at C₃, in connection with the above-mentioned turn structure R₁, an "L-based" structure of the inhibitor when bound to MMPs is obtained.

Most of the MMP inhibitors according to the state of the art are based on a succinyl basic structure and show a longer distance between the zinc binding region and C₃.

Therefore, R₂ binds to the S1' pocket, and there is no opportunity for the tail group to bind to this pocket. Therefore, the MMP inhibitors according to the state of the art, in contrast to the invention, bind in a substrate-like manner and therefore show an extended backbone in the MMP bound state.

The above-mentioned binding properties of the inhibitor can be determined using X-ray crystallographic techniques. Such methods are described e.g. by W. Bode et al., EMBO J. 13 (1994) 1263-1269 which is incorporated herein by reference for these techniques and for the crystal structure of the catalytic domain of HNC.

### Principle features of the MMP's catalytic domain

MMPs, e.g. HNC, exhibit a spherical shape, with a shallow active-site cleft separating a bigger "upper" N-terminal domain from a smaller "lower" C-terminal domain. The main upper domain consists of a central highly twisted five-stranded ,β-pleated sheet (with the β-strands ordered 2, 1, 3, 5, 4 and sheet strand 5 representing the only antiparallel strand), flanked by a double S-shaped loop and two other bridging loops on its convex side, and by two long α-helices including the active-site helix at its concave side.

Important substrate and inhibitor binding regions are the "edge" strand Leu(160) to Phe(164) of the β-sheet positioned "on top" of the active-site helix and forming the "northern" rim of the active-site cleft, and the preceding "bulged" segment Gly(155) to Leu(160), hereafter referred to as the "bulge segment", which is part of the S-shaped double-loop. The "catalytic" zinc ion is situated at the bottom of the active-site cleft and is coordinated to the Nε2 imidazole atoms of the three histidine residues of the His(197)-Glu(198)-X-X-His(201)X-X-Gly(204)-X-X-His(207) zinc-binding consensus sequence, and by one or two inhibitor atoms. In addition, the catalytic domain harbors a second "structural" zinc ion and two calcium ions packed against the top of the β-sheet.

The small lower domain consists of two concatenated wide loops and a C-terminal three-turn α-helix. The first of these wide right-handed loops includes a tight 1,4-turn stretching from Ala(213) to Tyr(216). This "Met-turn" represents a conserved topological element in the "metzincins"¹ providing a hydrophobic base for the three His residues, which ligate the catalytic zinc. The peptide chain then proceeds to the molecular surface at Pro(217) where the chain is linked and continues in an extended strand Pro(217)-Thr(224). The S1' pocket lies immediately to the "right" of the catalytic zinc and is formed by a long surface crevice (running perpendicular to the active-site cleft) separated from the bulk water by the initial part of this strand Pro(217)-Tyr(219) which forms its outer wall (referred to as "wall-forming segment"). The entrance to this pocket is formed by i) the bulge segment Gly(158)-Ile(159)-Leu(160) and the initial part of the edge strand Leu(160)-Ala(161) forming the "upper" side of the pocket, ii) the Tyr(219) side chain ("right" side), iii) the wall-forming segment including the Asn(218) side chain ("lower" side), and iv) the catalytic zinc together with the Glu(198) carboxylate group ("left" side). The features provide a series of polar groups for anchoring bound peptide substrates and inhibitors by hydrogen bonding (see below). The polar entrance bottleneck opens into the much more hydrophobic interior of the pocket bordered mainly by i) the side chains of Leu(160) and Val(194), ii) the Tyr(219) side chain, iii) the flat faces of the amide groups making up the wall-forming segment Pro(217)Tyr(219), and iv) the flat side of the imidazole ring of His(197). The inner part of the pocket is filled with 4 cross hydrogen bonded "internal" water molecules in addition to the 3 to 4 solvent molecules localized in the entrance of the pocket. The bottom of the pocket is partially secluded by the long side chain of Arg(222) which is flanked by the side chains of Leu(193) and Leu(214) and extends "behind"/"below" towards the Met-turn. The terminal guanidyl group is weakly hydrogen bonded to Pro(211)0, Gly(212)0 and/or Ala(213)0. Several interspersed polar groups provide anchoring points for the enclosed water molecules, one of which is in direct hydrogen bond contact with a localized bulk water molecule through an opening left between the Arg(222) side chain and the wall-forming segment.

### Binding of the inhibitors

Isolation, purification of the catalytic domain of HNC and crystallization and structural analysis of the catalytic domain of HNC with inhibitors and the binding of the state of the art inhibitors and inhibitors of the invention is described in WO 96/17838

### The synthesis of the inhibitors according to the invention

The manufacture of the inhibitors according to the invention can be carried out according to the methods known in the state of the art. As starting compounds, suitable malonic acid esters are used. For the substitution of the acidic hydrogen in R₂ position with larger alkyl or aryl groups, standard base catalyzed alkylation reactions of 1,3-dicarbonyl-CH acidic compounds (or alkylation of enolates) are used.
The hydroxamates are synthesized by acylating the protected hydroxylamine with the malonic acid derivatives, e.g. mixed anhydride, DCC (dicyclohexylcarbodiimide) or active esters, followed by deprotection.

The compounds of the present invention, which specifically inhibit MMPs, are pharmacologically useful in the treatment of rheumatoid arthritis and related diseases in which collagenolytic activity is a contributing factor, such as, for example, corneal ulceration, meningitis, osteoporosis, periodontitis, Paget's disease, gingivitis, tumor invasion, tumor metastasis, dystrophic epidermolysis, bullosa, systemic ulceration, epidermal ulceration, gastric ulceration, and the like. These compounds are particularly useful in the treatment of rheumatoid arthritis (primary chronic polyarthritis, PCP), systemic lupus erythematosus (SLE), juvenile rheumatoid arthritis, Sjogren's syndrome (RA + sicca syndrome), polyarthritis nodosa and related vasculitises, e.g. Wegener's granulomatosis, giant-cell arthritis, Goodpasture's syndrome, hypersensitiveness angiitis, polymyositis and dermatomyositis, tumor metastasis, tumor invasion, progressive system sclerosis, M. Behcet, Reiter syndrome (arthritis + urethritis + conjunctivitis), mixed connective tissue disease (Sharp's syndrome), spondylitis ankylopoetica (M. Bechterew).

The compounds of the present invention may be administered by any suitable route, preferably in the form of a pharmaceutical composition adapted to such a route and in dose effective for the treatment intended. Therapeutically effective doses of the compounds of the present invention required to prevent or arrest the progress of the medical condition are readily ascertained by one of ordinary skill in the art.
Accordingly, the invention provides a class of novel pharmaceutical compositions comprising one or more compounds of the present invention, in association with one or more non-toxic pharmaceutically acceptable carriers and/or dilutions and/or adjuvants (collectively referred to herein as "carrier materials") and, if desired, other active ingredients. The compounds and compositions may, for example, be administered intravascularly, intraperitoneally, subcutaneously, intramuscularly or topically.

For all administrations, the pharmaceutical composition may in the form of for example, a tablet, capsule, suspension or liquid. The pharmaceutical composition is preferably made in the form of a dosage unit contained in a particular amount of the active ingredient. Examples of such dosage units are tablets or capsules. A suitable daily dose for a mammal may vary widely depending on the condition of the patient and other factors. However, a dose of from about 0.1 to 300 mg/kg body weight, particularly from about 1 to 30 mg/kg body weight may be appropriate. The active ingredient may also be administered by injection.

The dose regimen for treating a disease condition with the compounds and/or compositions of this invention is selected in accordance with a variety of factors, including the type, age, weight, sex and medical conditions of the patient. Severity of the infection and the role of administration and the particular compound employed and thus may vary widely.

For therapeutic purposes, the compounds of the invention are ordinarily combined with one or more adjuvants appropriate to the indicated route of administration. If per os, the compounds may be admixed with lactose, sucrose, starch powder, cellulose esters of alkanoic acids, cellulose alkyl ester, talc, stearic acid, magnesium stearate, magnesium oxide, sodium and calcium salts of phosphoric and sulfuric acids, gelatin, acacia, sodium alginate, polyvinylpyrrolidone and/or polyvinyl alcohol, and thus tabletted or encapsulated for convenient administration. Alternatively, the compounds may be dissolved in water, polyethylene glycol, propylene glycol, ethanol, corn oil, cotton seed oil, peanut oil, sesam oil, benzyl alcohol, sodium chloride and/or various buffers. Other adjuvants and modes of administration are well and widely known in the pharmaceutical art. Appropriate dosages in any given instance, of course, depend upon the nature and severity of the condition treated, the route of administration and the species of mammal involved, including its size and any individual idiosyncrasies.
Representative carriers, dilutions and adjuvants include, for example, water, lactose, gelatin starch, magnesium stearate, talc, vegetable oils, gums, polyalkylene glycols, petroleum jelly, etc. . The pharmaceutical composition may be made up in a solid form, such as granules, powders or suppositories, or in liquid form, such as solutions, suspensions or emulsions. The pharmaceutical compositions may be subjected to conventional pharmaceutical operations, such as sterilization and/or may contain conventional pharmaceutical adjuvants, such as preservatives, stabilizers, wetting agents, emulsifiers, buffers, etc.

For use in the treatment of rheumatoid arthritis, the compounds of this invention can be administered by any convenient route, preferably in the form of a pharmaceutical composition adapted to such route and in a dose effective for the intended treatment. In the treatment of arthritis, administration may conveniently be by the oral route or by injection intra-articularly into the affected joint.

As indicated, the dose administered and the treatment regimen will be dependent, for example, on the disease, the severity thereof on the patient being treated and his response to treatment and, therefore, may be widely varied.

Within the sense of the present invention the following malonic acid based derivatives are preferred in addition to the compounds mentioned in the examples and compounds derived by combination of all meanings of substitutes mentioned in the claims:
1. N-Biphenyl-4-yl-N'-hydroxy-2-isobutyl-malonamide
2. N-Hydroxy-2-isobutyl-N'-(4-p-tolyloxy-phenyl)-malonamide
3. [4-(4-Chloro-phenoxy)-phenyl]-N'-hydroxy-2-isobutyl-malonamide
4. (2-Benzoylamino-4-phenoxy-phenyl)-N'-hydroxy-2-isobutyl-malonamide
5. N-Hydroxy-2-isobutyl-N'-(1-piperidin-1-ylmethyl-octyl)-malonamide
6. (1-Butyl-octyl)-N'-hydroxy-2-isobutyl-malonamide
7. [1-(2,2-Dimethyl-1-methylcarbamoyl-propylcarbamoyl)-octyl]-N'-hydroxy-2-isobutyl-malonamide
8. N-Hydroxy-2-isobutyl-N'-[1-(1-methylcarbamoyl-2-phenyl-ethylcarbamoyl)-octyl]-malonamide
9. N-Hydroxy-2-isobutyl-N'-(4-phenoxy-phenyl)-malonamide
10. N-Hydroxy-2-isobutyl-N'-(3-phenylpropyl)-malonamide
11. N-Hydroxy-2-isobutyl-N'-(5-phenyl-pentyl)-malonamide
12. N-Hydroxy-2-isobutyl-N'-octyl-malonamide
13. N-Decyl-N'-hydroxy-2-isobutyl-malonamide
14. N-Hydroxy-2-isobutyl-N'-(3-methyl-1-phenethyl-butyl)-malonamide
15. N-Hydroxy-2-isobutyl-N'-(1-isobutyl-5-phenyl-pentyl)-malonamide
16. 2-Benzyl-N-biphenyl-4-yl-N'-hydroxy-malonamide
17. 2-Benzyl-N-hydroxy-N'-(4-p-tolyloxy-phenyl)-malonamide
18. 2-Benzyl-N-[4-(4-chloro-phenoxy)-phenyl]-N'-hydroxy-malonamide
19. (2-Benzoylamino-4-phenoxy-phenyl)-2-benzyl-N'-hydroxy-malonamide
20. 2-Benzyl-N-hydroxy-N'-(1-piperidin-1-ylmethyl-octyl)-malonamide
21. 2-Benzyl-N-(1-butyl-octyl)-N'-hydroxy-malonamide
22. 2-Benzyl-N-[1-(2,2-dimethyl-1-methylcarbamoyl-propylcarbamoyl)-octyl]-N'-hydroxy-malonamide
23. 2-Benzyl-N-hydroxy-N'-[1-(1-methylcarbamoyl-2-phenyl-ethylcarbamoyl)-octyl]-malonamide
24. 2-Benzyl-N-hydroxy-N'-(4-phenoxy-phenyl)-malonamide
25. 2-Benzyl-N-hydroxy-N'-(3-phenyl-propyl)-malonamide
26. 2-Benzyl-N-hydroxy-N'-(5-phenyl-pentyl)-malonamide
27. 2-Benzyl-N-hydroxy-N'-octyl-malonamide
28. 2-Benzyl-N-decyl-N'-hydroxy-malonamide
29. 2-Benzyl-N-hydroxy-N'-(3-methyl-1-phenethyl-butyl)-malonamide
30. 2-Benzyl-N-hydroxy-N'-(1-isobutyl-5-phenyl-pentyl)-malonamide
31. N-Biphenyl-4-yl-N'-hydroxy-2-phenethyl-malonamide
32. N-Hydroxy-2-phenethyl-N'-(4-p-tolyloxy-phenyl)-malonamide
33. [4-(4-Chloro-phenoxy)-phenyl]-N'-hydroxy-2-phenethyl-malonamide
34. (2-Benzoylamino-4-phenoxy-phenyl)-N'-hydroxy-2-phenethyl-malonamide
35. N-Hydroxy-2-phenethyl-N'-(1-piperidin-1-ylmethyl-octyl)-malonamide
36. (1-Butyl-octyl)-N'-hydroxy-2-phenethyl-malonamide
37. [1-(2,2-Dimethyl-1-methylcarbamoyl-propylcarbamoyl)-octyl]-N'-hydroxy-2-phenethyl-malonamide
38. N-Hydroxy-N'-[1-(1-methylcarbamoyl-2-phenyl-ethylcarbamoyl)-octyl]-2-phenethyl-m
39. N-Hydroxy-2-phenethyl-N'-(4-phenoxy-phenyl)-malonamide
40. N-Hydroxy-2-phenethyl-N'-(3-phenyl-propyl)-malonamide
41. N-Hydroxy-2-phenethyl-N'-(5-phenyl-pentyl)-malonamide
42. N-Hydroxy-N'-octyl-2-phenethyl-malonamide
43. N-Decyl-N'-hydroxy-2-phenethyl-malonamide
44. N-Hydroxy-N'-(3-methyl-1-phenethyl-butyl)-2-phenethyl-malonamide
45. N-Hydroxy-N'-(1-isobutyl-5-phenyl-pentyl)-2-phenethyl-malonamide
46. N-Biphenyl-4-yl-N'-hydroxy-2-phenyl-malonamide
47. N-Hydroxy-2-phenyl-N'-(4-p-tolyloxy-phenyl)-malonamide
48. [4-(4-Chloro-phenoxy)-phenyl]-N'-hydroxy-2-phenyl-malonamide
49. (2-Benzoylamino-4-phenoxy-phenyl)-N'-hydroxy-2-phenyl-malonamide
50. N-Hydroxy-2-phenyl-N'-(1-piperidin-1-ylmethyl-octyl)-malonamide
51. (1-Butyl-octyl)-N'-hydroxy-2-phenyl-malonamide
52. [1-(2,2-Dimethyl-1-methylcarbamoyl-propylcarbamoyl)-octyl]-N'-hydroxy-2-phenyl-malonamide
53. N-Hydroxy-N'-[1-(1-methylcarbamoyl-2-phenyl-ethylcarbamoyl)-octyl]-2-phenyl-malonamide
54. N-Hydroxy-N'-(4-phenoxy-phenyl)-2-phenyl-malonamide
55. N-Hydroxy-2-phenyl-N'-(3-phenyl-propyl)-malonamide
56. N-Hydroxy-2-phenyl-N'-(5-phenyl-pentyl)-malonamide
57. N-Hydroxy-N'-octyl-2-phenyl-malonamide
58. N-Decyl-N'-hydroxy-2-phenyl-malonamide
59. N-Hydroxy-N'-(3-methyl-1-phenethyl-butyl)-2-phenyl-malonamide
60. N-Hydroxy-N'-(1-isobutyl-5-phenyl-pentyl)-2-phenyl-malonamide
61. 2-Benzyl-N-hydroxy-N'-(4-phenoxy-4-pyridyl)-malonamide
62. N-Hydroxy-2-isobutyl-N'-(4-phenoxy-4-pyridyl)-malonamide

The following examples and publications are provided to aid the understanding of the present invention, the true scope of which is set forth in the appended claims. It is understood that modifications can be made in the procedures set forth without departing from the spirit of the invention.

### Examples

### Synthesis of inhibitors

### Abbreviations:

- OSu:: N-Hydroxysuccinimide ester
- ONp:: p-Nitrophenylester
- Mal(iBu):: 2-Isobutyl-malonic acid
- Bn:: Benzyl
- Z:: Benzylcarboxy
- Boc:: t-Butylcarboxy
- homophe:: Homophenylalanine

### Example 1 (±)-HONH-Mal(iBu)-NH-(CH₂)₃-C₆H₅

### a. (±)-BnONH-Mal(iBu)-OH

To a suspension of O-benzylhydroxylamine hydrochloride (4.79 g; 30 mmol) in 50 ml THF sodium methylate (1.62 g 30 mmol) was added under stirring and after 10 min the solvent was evaporated. The residue was resuspended in 50 ml THF and reacted at 0 °C with (±)-EtO-Mal(*i*Bu)-OK (6.78 g; 30 mmol) and EDCI·HCl (6.34 g; 33 mmol). The reaction mixture was allowed to reach room temperature and after 12 h it was worked up by standard washings of the crude product in AcOEt with 5% KHSO₄, 5% NaHCO₃ and water. The organic layer was dried over MgSO₄, evaporated and the oily residue was purified by column chromatography on silica gel (0.040 - 0.063 mm) by elution with AcOEt/:n-hexane (1:2); yield of (±)-BnONH-Mal(*i*Bu)-OEt: 7.45 g (84.6 %) of colorless oil; TLC (AcOEt/*n*-hexane, 1:2; R_{f} 0.3); ¹H-NMR (d₆-DMSO): consistent with the structure.

(±)-BnONH-Mal(*i*Bu)-OEt (3.53 g; 12 mmol) was then saponified in 10 ml THF and 10 ml methanol with NaOH (1.44 g; 36 mmol) in 2 ml of water at 50 °C for 1 h. The reaction mixture was diluted with 50 ml methanol and desalted with 10 g amberlyst 15 (H⁺-form; 4.6 mmol/g) under ice-cooling. The resin was filtered off washed with methanol and the combined filtrates were evaporated to dryness; yield: 3.2 g (100 %) needles; TLC (CH₃CN/H₂0; 4:1): R_{f} 0.6; FAB-MS: m/z= 266.1 [M+1]⁺; Mᵣ=265.1 calcd. for C₁₄H₁₉NO₄; ¹H-NMR (d₆-DMSO): d= 11.26 (s, 1H, NHOBn), 7.34-7.39 (m, 5H, C₆H₅), 4.78 (s, 2H, CH₂ (Bn)), 3.03 (dd, 1H, H-C2 (Mal)), 1.37-1.65 (m, 3H, H-C3 (Mal) + H-C4 (Mal)), 0.85 (2*d, 6H, 2*CH₃).

### b. (±)-HONH-Mal(iBu)-NH-(CH₂)₃-C₆H₅

BnONH-Mal(iBu)-OH was converted to the (3-phenyl)propylamide as described for the phenetyl derivative and than hydrogenated under standard conditions to generate the title compound; Yield: 75 % over the 2 steps; mp 148 °C; ¹H-NMR (d₆-DMSO, 400 MHz): 10.46 (s, 1H, NHOH), 8.86 (s, 1H, NHOH), 7.69 (dd, 1H, NH-CH₂-CH₂-Bn)), 7.15 - 7.30 (m, 5H, C₆H₅), 3.09 (dd, 2H, NH-CH₂-CH₂-CH₂-Ph), 3.01 (t, 1H, H-C2(Mal)), 2.55 (dd, 2H, NH-CH₂-CH₂-CH₂-Ph), 1.69 (q, 2H, NH-CH₂-CH₂-CH₂-Ph, 1.58 (m, 2H, H-C3(Mal)), 1.43 (n, 1H, H-C4(Mal)), 0.86 + 0.84 (2* d, 6H, H-C5(Mal)).

### Example 2 (±)-HONH-Mal(iBu)-NH-(CH₂)₇-CH₃

The n-octylamide compound was prepared as described for the (3-phenyl)propyl derivative; Yield: 68.5 %; mp 151 °C; ¹H-NMR (d₆-DMSO, 400 MHz): 10.42 (s, 1H, NHOH), 8.86 (s, 1H, NHOH), 7.55 (dd, 1H, NH-C₈H₁₇), 3.03 (m, 2H, NH-CH₂-C₇H₁₅), 2.95 (t, 1H, H-C2(Mal)), 1.20 - 1.70 (m, 15H, 2 * H-C3(Mal), H-C4(Mal), NH-CH₂-C₆H₁₂-Me), 0.86 + 0.84 (m, 9H, NH-CH₂-C₆H₁₂-Me + H-C5(Mal)).

### Example 3 (±)-HONH-Mal(CH₂CH₂-C₆H₅)-NH-(CH₂)₃-C₆H₅

### a. (±)-EtO-Mal(CH₂CH₂-C₆H₅)-OEt

The title compound was obtained by alkylation of diethyl malonate with 2-phenylethylbromide according to standard procedures. The product was collected by distillation (bp. 122 °C; 0.1 mm); yield: 32.02 g (61 %) colorless oil; TLC (AcOEt/n-hexane, 1:1): Rf 0.7.

### b. (±)-BnONH-Mal(CH₂CH₂-C₆H₅)-OEt

(±)-EtO-Mal(CH₂CH₂-C₆H₅)-OEt was saponified with 1 equiv. KOH in EtOH at room temperature. The solvent was evaporated to dryness and the residue (2.74 g; 10.0 mmol) reacted with benzylhydroxylamine hydrochloride (1.60 g; 10,0 mmol), HOBT (1.5 g; 10 mmol), EDCI (1.92 g; 10 mmol) and NMM (1.01 g; 10 mmol) in THF (10 ml). After 12 h the reaction mixture was diluted with AcOEt and washed with 5 % KHSO₄, 5 % NaHCO₃ and water. The organic phase was dried over MgSO₄. The product was crystallized twice from AcOEt/n-hexane; yield: 1.87 g (55 %); TLC (AcOEt/n-hexane, 1:1): R_{f} 0.4; ¹H-NMR (d₆-DMSO) consistent with the assigned structure.

### c. (±)-HONH-Mal(CH₂CH₂-C₆H₅)-NH-(CH₂)₃-C₆H₅

(±)-BnONH-Mal(CH₂CH₂-C₆H₅)-OH was coupled with (3-phenyl)propylamine and worked up as described for the benzylamide derivative. Subsequent hydrogenation was performed in standard manner and the title compound was isolated in 70 % yield over the 2 steps; mp. 134 °C; ¹H-NMR (d₆-DMSO, 400 MHz): 10.46 (s, 1H, NHOH), 8.91 (s, 1H, NHOH), 7.72 (dd, 1H, NH-CH₂-CH₂-Bn)), 7.10 - 7.30 (m, 10H, 2 * C₆H₅), 3.09 (dd, 2H, NH-CH₂-CH₂-CH₂-Ph), 2.95 (t, 1H, H-C2(Mal)), 2.58 (t, 2H, H-C4(Mal)), 2.55 (t, 2H, H-C4(Mal)), 2.49 (dd, 2H, NH-CH₂-CH₂-CH₂-Ph),1.99 (q, 2H, H-C3(Mal)), 1.71 (q, 2H, NH-CH₂-CH₂-CH₂-Ph.

### Example 4 (±)-HONH-Mal(Bn)-NH-(CH₂)₃-C₆H₅

### a. (±)-BnONH-Mal(Bn)-OEt

EtO-Mal(Bn)-OEt was prepared as described for the phenethyl-derivative and isolated by destillation. The diethyl malonate derivative was saponified with KOH in EtOH at room temperature and the resulting KO-Mal(Bn)-OEt (3.90 g; 15.0 mmol) was reacted with benzylhydroxylamine-hydrochloride (2.40 g; 15,0 mmol), HOBT (1.5 g; 10 mmol), EDCI (3.10 g; 16 mmol) and NMM (1.51 g; 15 mmol) in THF. The reaction mixture was worked up as described for the phenethyl compound; yield: 4.3 g (88 %); TLC (AcOEt/n-hexane, 1:1): R_{f} 0.4; ¹H-NMR (d₆-DMSO): consistent with the structure.

### b. (±)-BnONH-Mal(Bn)-OH

(±)-BnONH-Mal(Bn)-OEt was saponified with KOH as described above for the phenethyl analog; yield: 95 %; TLC (CH₃CN/H₂O, 4:1): R_{f} 0.55.

### c. (±)-HONH-Mal(Bn)-NH-(CH₂)₃-C₆H₅

(±)-BnONH-Mal(Bn)-OH was converted to the (3-phenyl)propyl amide as described above for the phenethyl-analog and then hydrogenated in standard manner to produce the title compound in 60 % yield over the 2 steps; mp. 143 °C; ¹H-NMR (d₆-DMSO, 400 MHz): 10.41 (s, 1H, NHOH), 8.87 (s, 1H, NHOH), 7.73 (dd, 1H, NH-CH₂-CH₂-Bn)), 7.10 - 7.30 (m, 10H, 2 * C₆H₅), 3.05 (t, 1H, H-C2(Mal)), 3.04 (m, 4H, NH-CH₂-CH₂-CH₂-Ph, H-C3(Mal)), 2.50 (dd, 2H, NH-CH₂-CH₂-CH₂-Ph), 1.71 (q, 2H, NH-CH₂-CH₂-CH₂-Ph).

### Example 5

### Determination of the inhibitory effect (enzyme assay)

In order to determine the inhibition of MMPs, for example HNC, the catalytic domain (isolation and purification see example 1) is incubated with inhibitors having various concentrations. Subsequently, the initial reaction rate in the conversion of a standard substrate is measured in a manner analogous to F. Grams et al. (1993)³. The synthetic MMP substrate is a heptapeptide which is coupled, at the C-terminus, with DNP (Dinitrophenol). Said DNP residue quenches by steric hindrance the fluorescence of the adjacent tryptophane of the heptapeptide. After cleavage of a tripeptide which includes the DNP group, the tryptophane fluorescence increases. The proteolytic cleavage of the substrate therefore can be measured by the fluorescence value.
Assay buffer:
   50 mM Tris/HCl pH 7.6 (Tris = Tris-(hydroxymethyl)-aminomethan)
   100 mM NaCI 10 mM CaCl2 5% MeOH (if necessary)
Enzyme:
   8 nM catalytic domain (Met80-Gly242) of human neutrophil collagenase
Substrate:
   10 µM DNP-Pro-Leu-Gly-Leu-Trp-Ala-D-Arg-NH2
Total assay volume: 1 ml
   A solution of the enzyme and inhibitor in assay buffer (25°C) was prepared. The reaction was started directly by giving the substrate into the solution. The cleavage of the fluorogenic substrate was followed by fluorescence spectroscopy with an excitation and emission wavelength of 280 and 350 nm, respectively. The IC₅₀ value was calculated as the inhibitor concentration, which is necessary to decrease the velocity of the reaction to the half in comparison to the reaction without inhibitor.

**Table I**

| IC50 values for different inhibitors towards HNC | | |
|---|---|---|
| **Substance** | **Example number** | **IC**_{**50**} |
| HONH-(Mal(iBu)-homophe-NHhBn(p-Me) (of WO 96/17838) | | 1.6 µM |
| HONH-(Mal(iBu)-NH(CH₂)₃-Ph | 1 | 0.62 µM |
| HONH-COCH(Bn)-CO-NH(CH₂)₃-Ph | 4 | 0.64 µM |
| HONH-COCH(hBn)-CO-NH(CH₂)₃-Ph | 3 | 0.56 µM |
| HONH-(Mal(iBu)-NH-(CH₂)₇CH₃ | 2 | 0.30 µM |
| (Mal(iBu) = 2-isobutyl malonic acid; homophe = homophenyl alanine; NHhBn(p-Me) = 2-(4-methyl)phenylethylamine; hBn=homobenzyl; Bn=benzyl; Ph=phenyl). | | |

### References:

1) Bode, W., Gomis-Ruth, F.-X., Stocker, W., FEBS Lett. (1993) 331, 134-140.
2) Beckett RP, Davidson AH, Drummond AH, Huxley P, Whittaker M. Drug Disc. T. (1996)1: 16-26.
3) Grams, F. et al., FEBS 335 (1993)
4) Schechter, J., Berger, A., Biochem. Biophys. Res. Comm. (1967) 157-162.
5) Reinemer, P., Grams, F., Huber, R., Kleine, T., Schnierer, S., Pieper, M., Tschesche, H., Bode, W., FEBS L. (1994) 338, 227-233.
6) Lovejoy, B., Cleasby, A., Hassel, A.M., Longley, K., Luther, M.A., Weigl, D., McGeehan, G., McErroy, A.B., Drewry, D., Lambert, M.H., Jordan, S.R., Science 263 (1994) 375 377
7) Gooley, P.R., O'Connell, J.F., Marcy, A.I., Cuca, G.C., Salowe, S.P., Bush, B.L., Hermes, J.D., Esser, C.K., Hagmann, W.K., Springer, J.P., Johnson, B.A. Nature Struc. Biol. 1 (1994) 111-118

## Claims

1. A compound represented by general formula I, 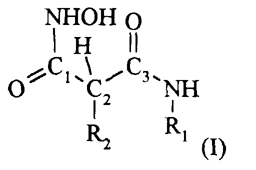 wherein
R₁ representing a chain which is optionally substituted once to four times and the chain consists of 7 to 11 backbone atoms comprising at least 7 C atoms with the proviso that no peptide groups are part of the chain and the proviso that at least the beginning three backbone atoms of the chain are carbon atoms,
R₂ is a residue of 2 to 10 backbone atoms, which interacts with the amino acid 161 of HNC, said residue being saturated or unsaturated, linear or branched, and contains optionally homocyclic or heterocyclic structures,
or pharmacologically acceptable salts or prodrugs therof.

2. A compound represented by general formula I, 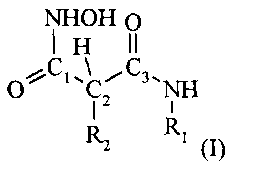 wherein
R₁ which binds into the S1 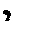 pocket and shows a logP-value between 1 and 4 if calculated as H₂N-R₁ with the proviso that no peptide groups are part of the chain and the proviso that at least the beginning three backbone atoms of the chain are carbon atoms,
R₂ is a residue of 2 to 10 backbone atoms, which interacts with the amino acid 161 of HNC, said residue being saturated or unsaturated, linear or branched, and contains optionally homocyclic or heterocyclic structures,
or pharmacologically acceptable salts or prodrugs therof.

3. Compounds of general formula I according to claim 1 or 2 wherein the chain in R₁ contains preferably no ester group.

4. Compounds of general formula I according to claim 1 wherein the number of substitutions of the chain is 2 or less and only one of the optional substitutes is within the first 3 backbone atoms.

5. Compounds of general formula I according to one of the claims 1 to 4 wherein R₁ is a residue which binds to the S1' pocket of HNC and is one of the following residues: 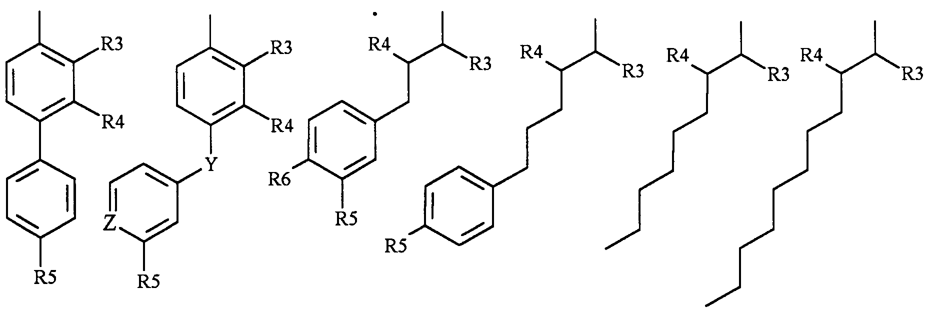 whereby
R3 and R4 independently have the meanings H, C₁-C₁₀-alkyl, aryl, arylalkyl, C₁-C₁₀-heteroalkyl, heteroaryl, heteroarylalkyl, heteroarylheteroalkyl, arylheteroalkyl,
R3 and R4 have in the case of a phenyl substitution in addition independently of each other the meaning of NR7R8
R7 and R8 is independently of each other hydrogen or lower alkyl
R3 and R4 have in the case of an alkyl substitution in addition independently of each other the meaning of SO₂R9 or COR10
R9 is hydrogen or lower alkyl
R10 is hydrogen, lower alkyl, or is one of the Cα-residues of an amino acid
R5 and R6 independently have the meanings H, OH, halogen, lower alkyl, lower alkenyl, or lower heteroalkyl, preferable at least one is hydrogen,
Y has the meanings O, S, NH, (CH₂)ₙ, n is 1, 2 or 3,
Z has the meanings N or CH.

6. Compounds of general formula I according to claim 5 wherein at least one of R3 and R4 is hydrogen, and at least one of R5 and R6 is hydrogen.

7. Compounds of general formula I according to one of the claims 1 to 6 wherein the maximal distance of an atom in R1 from the axis matching nitrogen and the last non hydrogen atom of the chain is 3 A.

8. Compounds of general formula I according to one of the claims 1 to 7 wherein the logP-value of R1 is between 2 and 3.2 if calculated as H₂N-R₁

9. Therapeutic composition comprising at least one of the compounds of formula I according to one of the claims 1 to 8 together with phamaceutical adjuvants and carrier.

10. Use of a compound of general formula I according of the claims 1 to 8 for the manufacture of a thearpeutic agent for the treatment of diseases in which MMP activity is a contributing factor
